(19) **Europäisches Patentamt**
**European Patent Office**
**Office européen des brevets**

(11) **EP 1 889 621 B1**

(12) **EUROPEAN PATENT SPECIFICATION**

(45) Date of publication and mention
of the grant of the patent:
**21.05.2014 Bulletin 2014/21**

(51) Int Cl.:
**A61K 31/485** *(2006.01)* **A61P 29/00** *(2006.01)*

(21) Application number: **07013702.1**

(22) Date of filing: **23.06.2004**

(54) **Multiparticulates**

Multipartikeln

Multiparticules

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(30) Priority: **27.06.2003 GB 0315137
12.02.2004 GB 0403102
16.06.2004 GB 0413454**

(43) Date of publication of application:
**20.02.2008 Bulletin 2008/08**

(62) Document number(s) of the earlier application(s) in
accordance with Art. 76 EPC:
**04743056.6 / 1 644 002**

(73) Proprietor: **EURO-CELTIQUE S.A.
1653 Luxembourg (LU)**

(72) Inventors:
• **Walden, Malcolm
Hardwick CB3 7XR
Cambridgeshire (GB)**
• **Hayes, Geoffrey Gerard
Saffron Walden CB10 1NA
Essex (GB)**
• **Mohammad, Hassan
Littleport CB6 1RS
Cambridgeshire (GB)**
• **Tamber, Harjit
Hitchin SG4 0BP
Hertfordshire (GB)**
• **Whitelock, Steve
Cambridge CB4 6ET
Cambridgeshire (GB)**
• **Martinelli, Vincenzo
Cambridge CB4 1SU
Cambridgeshire (GB)**

(74) Representative: **Williams, Gareth Owen et al
Marks & Clerk LLP
62-68 Hills Road
Cambridge
CB2 1LA (GB)**

(56) References cited:
**EP-A- 0 253 104     EP-A- 0 553 392
WO-A-01/58447     WO-A-93/10765
US-A- 5 958 452     US-A- 5 965 161**

Remarks:
The file contains technical information submitted after
the application was filed and not included in this
specification

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

## Description

The present invention relates to multiparticulates, and in particular to extruded multiparticulates which provide controlled release of oxycodone.

BACKGROUND OF THE INVENTION

Oxycodone is 4, 5-epoxy-14-hydroxy-3-methoxy-17-methylmorphinan-6-one and is derived from the opium alkaloid thebaine. It is a pure agonist opioid whose principal action is analgesia, and is usually administered as oxycodone hydrochloride. The hydrochloride salt of oxycodone is a white, odourless crystalline powder which dissolves freely in water (1 g in 6 to 7 ml).

Oxycodone is indicated for the treatment of moderate to severe pain. Controlled release oxycodone products enable management of pain when a continuous and around-the-clock supply of analgesic is needed for an extended period of time.

Formulations of oxycodone which provide controlled release of oxycodone are described for instance in WO 9310765. A granulation procedure is typically employed. In Example 3, a tablet containing 10 mg of oxycodone hydrochloride is prepared from a mix of oxycodone hydrochloride, lactose, povidone, Eudragit RS 30 D, triacetin, stearyl alcohol, talc and magnesium stearate. The same ingredients in adjusted amounts are employed in Example 4 to prepare tablets containing 20 mg oxycodone hydrochloride. The resultant products exhibit differing pharmacokinetic and pharmacodynamic properties.

Illustratively, the *in vitro* release rates of the 10 mg and 20 mg oxycodone tablets are given in WO 9310765 as follows:

|  | % oxycodone released | |
| --- | --- | --- |
| hour | 10mg | 20mg |
| 1 | 38.0 | 31 |
| 2 | 47.5 | 44 |
| 4 | 62.0 | 57 |
| 8 | 79.8 | 71 |
| 12 | 91.1 | 79 |
| 18 | 94.9 | 86 |
| 24 | 98.7 | 89 |

Tablets of this kind and with such release rates form the basis for a commercial product. Controlled release oxycodone tablets are available, as OxyContin (Registered Trade Mark) Tablets, which are designed to provide controlled delivery of oxycodone over 12 hours.

Oxycodone is well absorbed from OxyContin® Tablets with an oral bioavailability of 60% to 87%. The relative oral bioavailability of OxyContin® Tablets to immediate-release oral dosage forms is 100%. Upon repeated dosing in normal volunteers in pharmacokinetic studies, steady-state levels were achieved within 24-36 hours.

Dose proportionality has been established for 10 mg, 20 mg, 40 mg, 80 mg, and 160 mg tablet strengths with respect to both peak plasma levels ($C_{max}$) and extent of absorption (bioavailability), AUC, as indicated by the following data:

### Mean [% coefficient variation]

| Regimen | Dosage Form | AUC (ng.hr/mL)* | Cmax (ng/mL) | Tmax hrs) | Trough Conc. (ng/mL) |
| --- | --- | --- | --- | --- | --- |
| Single Dose | 10mg OxyContin® Tablets | 100.7 [26.6] | 10.6 [20.1] | 2.7 [44.1] | n.a. |

(continued)

| Regimen | Dosage Form | AUC (ng.hr/mL)* | Cmax (ng/mL) | Tmax hrs) | Trough Conc. (ng/mL) |
|---|---|---|---|---|---|
| | 20 mg OxyContin® Tablets | 207.5 [35.9] | 21.4 [36.6] | 3.2 [67.9] | n.a. |
| | 40 mg OxyContin® Tablets | 423.1 [33.3] | 39.3 [34.0] | 3.1 [77.4] | n.a. |
| | 80mg OxyContin® Tablets** | 1085.5 [32.3] | 98.5 [32.1] | 2.1 [62.3] | n.a. |
| Multiple Dose | 10mg OxyContin® Tablets q12h | 103.6 [38.6] | 15.1 [31.0] | 3.2 [69.5] | 7.2 [48.1] |
| | 5mg immediate release q6h | 99.0 [36.2] | 15.5 [28.8] | 1.6 [49.7] | 7.4 [50.9] |

*for single-dose AUC = $AUC_{0-inf}$, for multiple dose AUC = $AUC_{0-T}$
**data obtained while volunteers received naltrexone which can enhance absorption

Oxycodone is extensively metabolized and eliminated primarily in the urine as both conjugated and unconjugated metabolites. The apparent elimination half life of oxycodone following the administration of OxyContin® Tablets was 4.5 hours compared to 3.2 hours for immediate-release oxycodone.

About 60% to 87% of an oral dose of oxycodone reaches the central compartment in comparison to a parenteral dose. This high oral bioavailability is due to low pre-systemic and/or first-pass metabolism. In normal volunteers, the $t_{1/2}$ of absorption is 0.4 hours for immediate-release oral oxycodone. In contrast, OxyContin® Tablets exhibit a biphasic absorption pattern with two apparent absorption half-lives of 0.6 and 6.9 hours, which describes the initial release of oxycodone from the tablet followed by a prolonged release.

Alternative techniques exist for the manufacture of oxycodone formulations, apart from the granulation employed in the Examples of WO 9310765. Thus, multiparticulates of uniform dimensions with modified drug release properties can be manufactured by a technique referred to as melt extrusion technology. Melt extrusion is a solvent-free single-step process for manufacturing multiparticulates by extruding a softened blend, and is particularly useful for drug release modification. By selection of suitable polymers and additives, melt extrusion technology can be used both to enhance the solubility, and subsequently the bioavailability, of poorly water soluble drugs as well as to retard drug release of moderate to highly water soluble drugs for controlled release products.

The backbone of melt extrusion technology is the application of thermoplastic materials which act as binders for embedded drugs in solution or dispersion form within the matrix. Thermoplastic polymers with low glass transition temperatures (Tg) are preferred for processing by melt extrusion. Lower processing temperatures are also preferred with respect to the stability of heat sensitive drugs and other necessary excipients. Polymer glass transition temperatures can also be further reduced to facilitate processing at lower temperatures with optional addition of plasticisers.

Illustratively, WO 9614058 provides a sustained-release pharmaceutical formulation, comprising a melt-extruded blend of a therapeutically active agent, one or more materials selected from the group consisting of alkylcelluloses, acrylic and methacrylic acid polymers and copolymers, shellac, zein, hydrogenated castor oil, hydrogenated vegetable oil, and mixtures thereof; and one or more hydrophobic fusible carriers which provide a further retardant effect and are selected from the group consisting of natural or synthetic waxes, fatty acids, fatty alcohols, and mixtures thereof, the fusible carrier having a melting point from 30 to 200°C. The melt-extruded blend is divided into a unit dose containing an effective amount of said therapeutically active agent to render a desired therapeutic effect and providing a sustained-release of said therapeutically active agent for a time period of from about 8 to about 24 hours.

Furthermore, WO 9614058 describes a method of preparing a sustained-release pharmaceutical extrudate suitable for oral administration. The method comprises:

blending a therapeutically active agent together with (1) a material selected from the group consisting of alkylcelluloses, acrylic and methacrylic acid polymers and copolymers, shellac, zein, hydrogenated castor oil, hydrogenated vegetable oil, and mixtures thereof and (2) a fusible carrier selected from the group consisting of natural or synthetic waxes, fatty acids, fatty alcohols, and mixtures thereof; said retardant material having a melting point between 30-200°C and being included in an amount sufficient to further slow the release of the therapeutically active agent; heating said blend to a temperature sufficient to soften the mixture sufficiently to extrude the same; extruding said heated mixture as a strand having a diameter of from 0.1 -3 mm; cooling said strand; and dividing said strand to form non-, spheroidal multi-particulates of said extrudate having a length from 0.1-5 mm; and dividing said non-spheroidal multi-particulates into unit doses containing an effective amount of said therapeutically active agent, said unit dose providing a sustained-release of said therapeutically active agent for a time period of from about 8 to about 24 hours.

This method can be applied to oxycodone, an opioid analgesic, and typically employs a Eudragit polymethacrylate as the main retarding polymer in the matrix. The Eudragit polymethacrylates are widely employed in pharmaceutical compositions, notably to control release of an active ingredient. Thus, in some of the examples of WO 9614058, controlled release capsules or tablets with 20 mg of oxycodone hydrochloride are prepared by extrusion of a blend. In Examples 11 and 13, the oxycodone hydrochloride is blended with Eudragit RS PO, Eudragit L 100 and stearic acid. The blend in Example 12 additionally contains talc.

A need remains to provide a method of preparing multiparticulates of oxycodone which can be used to fill a capsule which can approximate to some or all of the pharmacokinetic and pharmacodynamic characteristics of Oxy-Contin® Tablets. A related object of this invention is the provision of a process for preparing an oxycodone pharmaceutical composition which provides an oxycodone *in vitro* release profile that approximates to that of Examples 3 and 4 of WO 9310765.

SUMMARY OF THE INVENTION

According to the present invention, we provide melt extruded controlled release multiparticulates which contain (a) oxycodone, (b) water-insoluble ammonium methacrylate copolymer, (c) plasticiser, (d) lubricant and (e) water permeability modifier, wherein said water permeability modifier is microcrystalline cellulose, croscarmellose sodium, crospovidone or sodium starch glycollate.

The present invention also provides a pharmaceutical composition in unit dose form comprising multiparticulates as herein before defined.

We provide oxycodone multiparticulates with a high initial release of oxycodone, and a high total release of oxycodone. The release properties can the expressed in forms of release of oxycodone under controlled *in vitro* conditions which for example simulate human gastric fluids or the human intestinal environment. Release at a physiological pH, for example a pH of about 1.2 or about 6.8, can be tested. Test procedures can also be designed to reflect a switch from the stomach to the intestins during passage through the body.

In particular, we have found that the inclusion of a water permeability modifier can permit extrusion of multiparticulates of oxycodone which show some bioequivalence to OxyContin® Tablets. The multiparticulates can have pharmacokinetic and/or pharmacodynamic properties approximating to these of OxyContin® Tablets. In particular, the multiparticulates can have *in vitro* release rates that approximate to these of OxyContin® Tablets.

In the invention, we provide oxycodone multiparticulates comprising oxycodone usually in the form of a pharmaceutically acceptable salt, a water-insoluble ammonium methacrylate copolymer, a plasticiser, a lubricant and a water permeability modifier as specified above. Typically the water permeability modifier serves to modify the water permeability and enhance the drug release, especially in the later stages of the dissolution. The water permeability modifier can also serve to modulate the rate of secretion of the drug.

The oxycodone can be in the form of a pharmaceutically acceptable salt, preferably the hydrochloride, or the free base.

The multiparticulates are obtainable by melt extrusion of an extrudable blend. Such an extrusion is disclosed in WO 9614058 and referred to as a melt extrusion. In practice, the polymer softens but in practice might not melt.

The multiparticulate of this invention can be used as a fill in a capsule. Thus, the present invention provides a capsule suited for once or twice a day dosing. Other dosage forms of the controlled release , formulation can be provided. The dosage form is preferably a unit dosage form, and preferably shows some bioequivalence to OxyContin® Tablets. The dosage form can have pharmacokinetic and/or pharmacodynamic properties approximating to those of OxyContin® Tablets. In particular, the dosage form can have *in vitro* release rates that approximate to those of OxyContin® Tablets.

The invention also provides a composition as defined herein for use in providing pain relief or analgesia.

The invention also provides multiparticulates as defined herein for use in providing pain relief or analgesia.

The invention also provides the use of multiparticulates as herein defined in the preparation of a composition for use in providing pain relief or analgesia.

In a related aspect, we also provide a process for preparing multiparticulates as claimed in claim 1 which comprises preparing a blend which contains (a) oxycodone, (b) water-insoluble ammonium methacrylate copolymer, (c) plasticiser, (d) lubricant and (e) water permeability modifier, wherein said water permeability modifier is a microcrystalline cellulose, croscarmellose sodium, crospovidone or sodium starch glycollate; and extruding the blend.

DETAILS OF THE INVENTION

The oxycodone multiparticulates of this invention preferably give *in vitro* release rates that approximate to those of OxyContin® Tablets. The release rates of OxyContin® Tablets are notable for a high initial release, and a high total release. Preferably the release of oxycodone is substantially independent of pH in the pH range of around 1 to around 7. To this end, substantially pH-independent release can mean that for a given formulation when tested in simulated intestinal fluid at pH 6.8, at any given time point the amount of oxycodone released as a percentage of the original amount of oxycodone in the formulation is substantially equal to the percentage amount of oxycodone released based on the original amount of oxycodone in the formulation when tested in simulated gastric fluid at pH 1.2. The release is substantially equal when the respective amounts differ by ± 30%, more preferably ± 20% and most preferably ± 15%.

Unless otherwise indicated, we measure release rates by a specified method which involves using Ph.Eur. basket dissolution apparatus at 37°C, 100 rpm in 900 ml of USP simulated gastric fluid at pH 1.2 without enzyme. In one variation, the dissolution medium is simulated intestinal fluid at pH 6.8 without enzyme.

For simulated gastric fluid at pH 1.2, the oxycodone multiparticulates of this invention typically release at least 15% oxycodone after 1 hour, reflecting a high initial release. Preferably they release at least 20%, more preferably at least 25% and most preferably at least 35% of the oxycodone after 1 hour.

The oxycodone multiparticulates of this invention typically release at least 30% oxycodone after 2 hours, reflecting a high initial release. Preferably they release at least 40%, more preferably at least 50% and most preferably at least 55% of the oxycodone after 2 hours.

The oxycodone multiparticulates of this invention typically release at least 60% oxycodone after 4 hours, reflecting a high initial release. Preferably they release at least 70%, more preferably at least 75% and most preferably at least 80% of the oxycodone after 4 hours.

The oxycodone multiparticulates of this invention typically release at least 75% oxycodone after 10 hours, reflecting a high total release. Preferably they release at least 80%, more preferably at least 90% and most preferably at least 95% of the oxycodone after 10 hours.

Furthermore, at least 85% release of oxycodone after 8 hours is preferred. The oxycodone multiparticulates of this invention can release 100% oxycodone after 12 hours, reflecting a high total release.

The multiparticulates of this invention contain (a) Oxycodone, (b) water-insoluble ammonium methacrylate copolymer, (c) plasticiser, (d) lubricant and (e) water permeability modifierwherein said water permeability modifier is microcrystalline cellulose, croscarmellose sodium, crospovidone or sodium starch glycollate. With this selection of ingredients it becomes possible to prepare multiparticulates and thus capsules containing oxycodone and which mimic the *in vitro* and preferably the *in vivo* release characteristics of OxyContin® Tablets. In particular, the combination including a water permeability modifier enables an adequate initial release of oxycodone (early hours) whilst maintaining a high total release of the active ingredient in the later hours of dissolution.

Oxycodone hydrochloride is the preferred form of oxycodone, though other pharmaceutically acceptable salts can be used.

The water-insoluble ammonium methacrylate copolymer, also referred to as a water-insoluble ammonio methacrylate copolymer, is suitably Eudragit RS PO. It offers the following properties:

- ○ insoluble to poorly water soluble,
- ○ low aqueous porosity or permeability,
- ○ compatible with the drug and other additives,
- ○ extrudable at moderate temperatures or at lower temperatures in the presence of a suitable plasticiser,
- ○ stable for the intended storage time and conditions,
- ○ thermal stability.

In particular, Eudragit RS PO is a thermoplastic polymer of low water permeability which can significantly retard release of embedded oxycodone in its matrix. It is described as a pH independent polymer powder with low permeability for matrix formulations. It is a copolymer of acrylic and methacryrylic acid esters, with a low content of quaternary ammonium groups to control permeability, and an average molecular weight of around 150,000.

The plasticiser serves to soften the insoluble ammonium methacrylate copolymer to make it more easy to extrude the polymer. To this end, the typical plasticiser is miscible with the insoluble ammonium methacrylate copolymer to produce a decreased tensile strength, a lower softening temperature, and a decrease in the glass transition temperature, Tg, of the polymer. It serves to reduce cohesion by providing internal lubrication of the polymer. The plasticiser is normally chosen from water insoluble solids such as cetyl alcohol, stearyl alcohol and cetostearyl alcohol; water soluble solids such as sorbitol and sucrose and high molecular weight polyethylene glycol; water insoluble liquids such as dibutyl sebacate and tributyl citrate and water soluble liquids such as triethyl citrate, propylene glycol and low molecular weight polyethylene glycol. Stearyl alcohol is a preferred plasticiser. Another preferred plasticiser is a high molecular weight polyethylene glycol, preferably with a molecular weight in the range 4000 to 10000, such as PEG 6000.

The lubricant is a processing aid which reduces friction between the plasticised polymer blend and the internal surfaces of the extruder. It is normally a solid, and is suitably chosen from stearic acid, glyceryl behenate (predominantly glyceryl dibehenate), magnesium stearate, calcium stearate, talc and silicon dioxide (fused silica). The presence of lubricant in the extrusion formulation improves blending, kneading and conveying, and reduces adhesion forces. Smooth lubricated extrusion at low to moderate temperatures improves batch to batch reproducibility and reduces the strain on both the product and equipment. Stearic acid, possibly in the form of a salt, is a preferred lubricant. Another preferred lubricant is glyceryl behenate, which give less pH sensitivity for *in vitro* release of oxycodone.

Plasticisers can often act as a lubricant, and lubricants can often act as a plasticiser.

The choice of plasticiser and lubricant will usually have an effect on the characteristics of the resultant extruded multiparticulates. For example, where the plasticiser is stearyl alcohol and the lubricant is stearic acid, the quantities and ratios with respect to each other and relative to the ammonium methacrylate copolymer can significantly modify the release rate of the drug. We have found that higher levels of stearyl alcohol reduce the Tg of the polymer blend and believe this reduction affects the rate of drug release. However, higher levels of stearic acid can also improve the mixing, kneading and extrusion as well as alter the release rate of oxycodone. We have found that higher ratios of stearic acid at only the expense of stearyl alcohol show a significant reduction of the rate and total oxycodone release.

The water permeability modifier modulates secretion of the drug from the dosage form. Typically the water permeability modifier serves to enhance the drug release, especially in the later stages of the dissolution, though we also envisage that the water permeability modifier might in some instances play a role in slowing release. Examples of agents used to modify the water permeability of the extruded multiparticulates include an insoluble hydrophilic wicking agent.

For example, microcrystalline cellulose may be used to enhance the total release of the active.

Microcrystalline cellulose improves water diffusion and exchange and thus enhances drug release. The microcrystalline cellulose acts as an insoluble but hydrophilic wicking agent. Alternatives to microcrystalline cellulose are croscarmellose sodium, crospovidone or sodium starch glycollate.

Suitable percentage amounts for the ingredients (a) to (e) are given in the following table, based on the total weight of the five ingredients:

|  | typical range % | preferred range % | more preferred range % |
| --- | --- | --- | --- |
| oxycodone as hydrochloride | 3 to 50 | 5 to 40 | 7.5 to 35 |
| insoluble ammonium methacrylate copolymer | 25 to 85 | 35 to 75 | 50 to 65 |
| plasticiser | 1 to 30 | 3 to 25 | 5 to 15 |
| lubricant | 1 to 25 | 2 to 25 | 2 to 25 |
| water permeability modifier | 1 to 40 | 1 to 30 | 1 to 20 |

Other additives may also be employed to produce multiparticulates within a set of predetermined specifications. Bulking agents, for example lactose, microcrystalline cellulose and calcium phosphate, are widely used pharmaceutical excipients and can be used in the present invention to modify the release rates and/or total release. Other release modifying agents may also be considered to modulate the release rate and/or enhance total release.

The preferred formulation contains oxycodone, preferably as the hydrochloride salt, Eudragit RS PO as water-insoluble ammonium methacrylate copolymer, stearyl alcohol as plasticiser, glyceryl behenate as lubricant, and microcrystalline cellulose as water permeability modifier.

For manufacture or the multiparticulates of this invention, the ingredients are blended, and extruded. Details of such procedures are given in WO 9614058.

For the present invention, we prefer to employ a twin screw extruder, which can have co-rotating or counter-rotating screws. Essentially, the blend as a powder is fed by a feeder into the first segment of the barrel usually at relatively low temperature, for example 10-20°C, to ensure a constant powder flow to the high temperature barrels. The feeder provides a uniform current of the blend to the extruder. Consistency is desirable as irregular and variable feeding rates can produce multiparticulates with varying physical properties, such as density and porosity.

The preferred extruder is designed with twin screws, preferably counter-rotating screws, for the task of conveying, blending, compressing, heating and softening the blend. Depending on the choice of the components of the blend and the extrusion conditions, it may be that the blend will melt as well as soften. The screws which perform a significant part of this extrusion process are built of different smaller elements chosen from a variety of screw elements and kneader elements. Mixing and kneading time can be significantly altered by changing the type, length and configuration of the screw elements and possibly kneader elements. Short residence times and moderate to low shear forces contribute to safe processing and stable product even with heat sensitive drugs. Examples of available extruders include those manufactured by Leistritz, Brabender, Randcastle, and Kurimoto Co. Ltd.

Screw rotating speeds may play a part in the quality of the multiparticulates produced. High rotation speeds without appropriate compensation of the blend feed rate may produce high porosity multiparticulates with a variable drug release rate. On the other hand slow screw rotation would induce unnecessary long residence times. A vacuum connected to the extruder barrel is desirable to remove trapped air within the softened blend and thus produce dense non-porous multiparticulates.

The extrusion head is typically designed to produce multiple strands of fixed diameter. The number, shape and diameter of the orifices can be changed to suit a predetermined specification.

In addition to the screw speed, the other main influential parameters are the screw torque, individual barrel temperature, and extrusion head pressure and temperature.

In accordance with one cutting procedure of this invention, the extruded strands are carried away from the die-head on a conveyer. The strand diameter is affected by the blend feed rate, die-head orifice diameter, screw speed, barrel temperature, nip rolls speed and conveying speed. Conveying is appropriate to carry the extruded strand to a laser gauge or other measuring device to achieve a desired diameter such as 1.0 mm. During this conveying process the strands cool down gradually, but essentially remain flexible. Flexible strands retain integrity on the laser gauging device, between the pelletiser feed nip rolls and during entry to the pelletiser. Rapidly cooled strands, depending on the formulation, may lose their integrity and shatter during passage through the nip rolls and pelletiser into uneven-shaped and irregular-sized multiparticulates.

The strands are fed into the pelletiser by nip rolls. The pelletiser cuts the fed strands, for instance using a rotary knife cutter, to a predetermined length, for example 1.0 mm. The feeding rate of the strands and the pelletiser cutter speed determine the length of the multiparticulates,

Overall, the co-ordination/interaction between the powder feeder, extruder, conveyor, laser gauge and pelletiser is an important parameter affecting the quantity, quality and reproducibility of the final multiparticulate products.

Multiparticulates produced by this cutting procedure where the extruded strands are carried away from the die-head typically take the form of cylinders.

In another preferred cutting procedure, a cutter cuts the extruded mix as it emerges under pressure and still softened from the orifices of the die plate. The cutter is suitably a rotary cutter with one or more blades which sweep over the surface of the die-head to pass the orifices. Two diametrically opposed blades are preferred. Ideally, the inner and outer surface boundaries to the extrusion orifices are coated with a non-stick material, e.g. a polytetrafluoroethylene (PTFE). As the cut extrudate particles expand and cool, they tend to form rounded surfaces. By appropriate adjustment of the extrusion pressure, the rate of extrusion and the speed of the cutter blade, it is possible to arrange for spherical or near-spherical multiparticulates to be obtained. Alternatively, this process can be operated to produce rods if desired. In one embodiment a stream of air is directed at the surface of the die-head, the air being at a reduced temperature to cool the extrudate and speed solidification.

Spherical multiparticulates produced by this method offer a number of possible advantages:

> Better batch to batch reproducibility.
> Easier coating and lower coating weight required.
> Better capsule filling and higher yield.
> More stable at elevated temperature.
> More tamper resistant.
> Reduced downstream processing.
> Reduce or eliminate some problems that arise during conveying and pelletising the strands such as strands shattering to different length pellets and static charge.

The multiparticulates may be divided into unit doses such that each individual unit dose includes a dose of oxycodone sufficient to provide analgesia to a mammal, preferably a human patient. A suitable dose of oxycodone is 5 to 400 mg, especially 5 mg, 10 mg, 20 mg, 40 mg, 80 mg or 160 mg unit dosages. In this respect, a unit dose contains an effective amount of the therapeutically active agent to produce pain relief and/or analgesia to the patient. The dose of oxycodone administered to a patient will vary due to numerous factors, including the weight of the patient, the severity of the pain, the metabolic status and the nature of any other therapeutic agents being administered.

In one preferred embodiment, the multiparticulates are filled into hard gelatin capsules each containing a unit dose. The fill weight in the capsule is preferably in the range 80 to 500 mg, more preferably 120 to 500 mg, In a variation of this invention, the unit doses of multiparticulates may be incorporated into other solid pharmaceutical dosage formulations, for example using compression or shaping into tablets, or by forming the extruded product into the form of a suppository.

The capsules or other unit dose forms of this invention preferably are designed for administration at intervals of about 12 hours. To this end, the unit dose form suitably has an oxycodone dissolution rate *in vitro*, when measured by the USP Paddle Method (see the U.S. Pharmacopocia XXII 1990) at 100 rpm in 900 ml aqueous buffer (pH between 1.6 and 7.2) at 37°C of between 12.5 and 42.5% (by wt) oxycodone released after 1 hour, between 25 and 56% (by wt) oxycodone released after 2 hours, between 45 and 75% (by wt) oxycodone released after 4 hours and between 55 and 85% (by wt) oxycodone released after 6 hours. Furthermore, we prefer that the peak plasma level of oxycodone obtained *in vivo* occurs between 2 and 4.5 hours after administration of the dosage form.

More information on desirable characteristics for such oxycodone formulations is given in WO 9310765.

Using our specified method at pH 1.2, simulated gastric fluid, the release rates are suitably as follows:

Preferred Limits

| Hour | % Released Lower Limit | % Released Upper Limit |
|------|------------------------|------------------------|
| 1 | 16 | 56 |
| 2 | 37 | 77 |
| 4 | 60 | 100 |
| 10 | 75 | 100 |

More Preferable Limits

| Hour | % Released Lower Limit | % Released Upper Limit |
|------|------------------------|------------------------|
| 1 | 21 | 51 |
| 2 | 42 | 72 |
| 4 | 65 | 95 |
| 10 | 80 | 100 |

Most Preferred Limits

| Hour | % Released Lower Limit | % Released Upper Limit |
|------|------------------------|------------------------|
| 1 | 24 | 48 |
| 2 | 45 | 69 |
| 4 | 68 | 92 |
| 10 | 83 | 100 |

Using our specified method at pH 6.8, simulated intestinal fluid, the release rates are suitably as follows:

Preferred Limits

| Hour | % Released Lower Limit | % Released Upper Limit |
|------|------------------------|------------------------|
| 1 | 11 | 51 |
| 2 | 28 | 68 |

(continued)

| Hour | % Released Lower Limit | % Released Upper Limit |
|------|------------------------|------------------------|
| 4 | 48 | 88 |
| 10 | 61 | 100 |

More Preferable Limits

| Hour | % Released Lower Limit | % Released Upper Limit |
|------|------------------------|------------------------|
| 1 | 16 | 46 |
| 2 | 33 | 63 |
| 4 | 53 | 83 |
| 10 | 65 | 96 |

Most Preferred Limits

| Hour | % Released Lower Limit | % Released Upper Limit |
|------|------------------------|------------------------|
| 1 | 19 | 43 |
| 2 | 36 | 60 |
| 4 | 56 | 80 |
| 10 | 69 | 93 |

As an alternative to administration at intervals of about 12 hours, the capsules or other unit dose forms of this invention are designed for administration at intervals of about 24 hours. To this end, the unit dose form suitably has an oxycodone dissolution rate *in vitro*, when measured by the USP Basket Method at 100 rpm in 900 ml aqueous buffer at a pH between 1.6 and 7.2 at 37°C of from 0% to about 40% at 1 hour, from about 8% to about 70% at 4 hours, from about 20% to about 80% at 8 hours, from about 30% to about 95% at 12 hours, from about 35% to about 95% at 18 hours, and greater than about 50% at 24 hours. Furthermore, we prefer that the peak plasma level of oxycodone obtained *in vivo* is reached at about 2 hours to about 17 hours after administration at steady state of the dosage form.

More information on desirable characteristics for such oxycodone formulations is given in WO 02087512;

In a variation, the present invention provides unit doses which contain oxycodone and an oxycodone antagonist effective to prevent tampering. In this respect, reference is made to WO 0313433.

In particular, the unit dose can contain oxycodone and naltrexone. Other opioid antagonists which are known in the art can be used, for example naloxone.

The present invention provides extruded multiparticulates of oxycodone, and extruded multiparticulates of oxycodone antagonist such as naltrexone. The naltrexone multiparticulates do not release naltrexone on conventional administration, and for example have a non-release coating. Both populations are preferably visual and physically identical.

An important aspect of this invention is a capsule with a unit dose fill of less than 500 mg, comprising up to about 350 mg of oxycodone multiparticulates, and up to about 200 mg of tamper-proof oxycodone antagonist multiparticulates. For example, there can be 120 to 300 mg of oxycodone multiparticulates, and 125 to 175 mg of tamper-proof oxycodone antagonist multiparticulates.

## EXAMPLES OF THE INVENTION

### Standardised Conditions

For the following experimental work, standardised conditions were established for the extrusion of oxycodone hydrohloride blends. The extruder was a Leistritz 18 at 140 rpm, with a feed rate of 2.6 kg/h producing pellets of 1 mm diameter and 1 mm length.

The design of the screw uses components of the distributor Leistritz USA. The aim is to optimise the mixture by adding extra mixing elements 'GGC2' or 'ZS' to avoid mixing problems, and to increase the residence time by including 'FD' elements to avoid wetting problems

The extruder comprises ten zones, with zone 1, zone 2 and so on up to zone 8, and then zones 9 and 10 are at the extruder head.

Typical batch zone temperatures were as follows (°C):

| Example | 1 | 2 | 3-6 | 7-8 | 9 | 10 | Melt pressure (bar) | Torque (%) |
|---|---|---|---|---|---|---|---|---|
| 5 | 14 | 40 | 90 | 75 | 83 | 90 | 63-68 | 53-59 |
| 8 | 14 | 40 | 90 | 75 | 65 | | 61-62 | 49 |
| 9 | 14 | 40 | 125 | 120 | 125 | 125 | 99-107 | 78-84 |
| 10 | 14 | 40 | 120 | 105-106 | 115 | 120 | 73-77 | 174-79 |
| 11 | 14 | 40 | 101-103 | 100 | 106 | 106 | 99-115 | 89-97 |

For Examples 9 to 11, the temperatures were raised significantly The feed rate and screw speed were generally kept constant although the conveyor speed, nip rolls speed and pelletiser speed changed according to the properties of the extrudate when it emerged from the die plate (this was highly dependent on the way the extrudate expanded and hence hard to correlate to previous batches).

Two drug loads (8.3 and 25% by weight) of oxycodone extruded multiparticulate formulations (see tables) were planned to cover doses of 10 mg and 40 mg.

For the 8.3% oxycodone load, the following trial batches were prepared, where the weights are mg per unit dose. Examples 3, 4, 5, 7, 8, 9, 10 and 11 are outside the scope of the invention.

| | Example | | | |
|---|---|---|---|---|
| | 1 (Comparative) | 2 | 3 | 4 |
| Oxycodone HCl | 10 | 10 | 10 | 10 |
| Eudragit RS PO | 77 | 72 | 62 | 74 |
| Stearyl alcohol | 24.75 | 24 | 24 | 24 |
| Stearic acid | 8.25 | 4 | 4 | 4 |
| Microcrystalline cellulose (Avicel PH 101) | | 10 | | |
| Eudragit RL PO | | | 20 | 8 |
| Hydroxypropylmethyl cellulose (HPMC K100M) | | | | |

| Total | 120 | 120 | 120 | 120 |
|---|---|---|---|---|

| | Example | | | |
|---|---|---|---|---|
| | 5 | 6 | 7 | 8 |
| Oxycodone HCl | 10 | 10 | 10 | 10 |
| Eudragit RS PO | 77 | 69 | 74 | 70 |
| Stearyl alcohol | 24 | 24 | 16 | 16 |
| Stearic acid | 4 | 4 | 12 | 12 |
| Microcrystalline cellulose (Avicel PH101) | | 13 | | |
| Eudragit RL PO | 6 | | | |
| Hydroxypropylmethyl cellulose (HPMC K100M) | | | 8 | 12 |
| Total | 120 | 120 | 120 | 120 |

| | Example | | |
|---|---|---|---|
| | 9 | 10 | 11 |
| Oxycodone HCl | 10 | 10 | 10 |
| Eudragit RS PO | 68 | 66 | 74 |
| Stearyl alcohol | 8 | 14 | 14 |
| Eudragit RL PO | 28 | 25 | 17 |
| Glyceryl behenate | 6 | 5 | 5 |
| Total | 120 | 120 | 120 |

For the 25% oxycodone load, the following trial batches were prepared, where the weights are mg per unit dose. Examples 14, 15, 18 and 19 are outside the scope of the invention

| | Example | | | | |
|---|---|---|---|---|---|
| | 12 Comparative | 13 Comparative | 14 | 15 | 16 |

| Oxycodone HCl | 40 | 40 | 40 | 40 | 40 |
|---|---|---|---|---|---|
| Eudragit RS PO | 90 | 90 | 85 | 87 | 82 |
| Stearyl alcohol | 10 | 20 | 20 | 20 | 20 |
| Stearic acid | 20 | 10 | 10 | 10 | 10 |
| Eudragit RL PO | | | 5 | 3 | 8 |
| Total | 160 | 160 | 160 | 160 | 160 |

| | Example | | |
|---|---|---|---|
| | 17 | 18 | 19 |
| Oxycodone HCl | 40 | 40 | 40 |
| Eudragit RS PO | 78 | 82 | 78 |
| Stearyl alcohol | 20 | 8 | 8 |
| Stearic acid | 10 | 22 | 22 |
| Microcrystalline cellulose (Avicel PH101) | 12 | | |
| Hydroxypropylmethyl cellulose (HPMC K100M) | | 8 | 12 |
| Total | 160 | 160 | 160 |

Release rate studies

The oxycodone extruded multiparticulates of Examples 1 to 19 were tested for dissolution using Ph.Eur. basket dissolution apparatus at 37°C, 100 rpm in 900 ml of USP simulated gastric fluid at pH 1.2 without enzyme. Standard HPLC procedures were used for assay.

Additionally, the oxycodone extruded multiparticulates of Example 9 were tested for dissolution using Ph.Eur. basket dissolution apparatus at 37°C, 100 rpm in 900 ml of simulated intestinal fluid at pH 6.8 without enzyme, Again, standard HPLC procedures were used for assay.

The *in vitro* release rates were measured, and plotted.

Microcrystalline cellulose

10 and 13 mg/120 mg oxycodone extruded multiparticulates and 8 and 12 mg/ 160 mg oxycodone extruded multiparticulates were used in the 8.3% and 25% oxycodone hydrochloride loaded formulations respectively.

**Glyceryl behenate**

**Dissolution data for the formulations of Examples 9 to 11** demonstrates that the inclusion of glyceryl behenate can give the desired high initial release combined with high total release.

Bioavailability study

The formulations of Examples 2, 5 and 8 were investigated along with OxyContin® Tablets in a Phase I bioavailability study, where they were identified respectively as B, A and C. The study was a four-period randomised incomplete block crossover study, involving 24 healthy male and female subjects. A single dose of 2 x 10mg capsules (20mg total) of Example 2, Example 5, Example 8 or a 20 mg OxyContin® Tablet was administered to the subjects. Each test formulation was administered after an overnight fast, or following ingestion of a high fat breakfast.

The mean parameters from this study are summarised in the following table. Examples 5 and 8 are outside the scope of the invention.

|  | Example 5 fasted. (n = 13) | Example 5 fed (n = 13) | Example 2 fasted (n = 11) | Example 2 fed (n = 14) |
|---|---|---|---|---|
| AUCt (ng.h/mL)* | 223.2 | 272.4 | 212.2 | 255.5 |
| SD | (47.07) | (76.93) | (48.49) | (44.91) |
| $AUC_{INF}$ (ng.h/mL)* | 231.9 | 277.7 | 220.3 | 261.3 |
| SD | (46.16) | (77.27) | (51.54) | (45.83) |
| $C_{max}$ (ng/mL)* | 21.6 | 26.9 | 15.4 | 21.5 |
| SD | (5.07) | (6.78) | (2.81) | (4.12) |
| $t_{max}$ (h)** | 3.0 | 5 | 3 | 5 |
| Range | (2-6) | (2.5-5) | (2-5) | (3-6) |
| * arithmetic mean ** median | | | | |

|  | Example 8 fasted (n =14) | Example 8 fed (n = 12) | OxyContin® Tablets (n = 13) |
|---|---|---|---|
| AUCt (ng.h/mL)* | 232.9 | 298.19 | 210.6 |
| SD | (45.32) | (51.63) | (33.07) |
| $AUC_{INF}$ (ng.h/mL)* | 239.6 | 302.3 | 212.6 |
| SD | (44.90) | (53.63) | (32.76) |
| $C_{max}$ (ng/mL)* | 12.4 | 20.0 | 19.1 |
| SD | (3.52) | (3.73) | (4.34) |
| $t_{max}$ (h)** | 3.6 | 5 | 2.5 |
| Range | (2-6) | (5-8) | (1.5-5) |
| * arithmetic mean **median | | | |

With the exception of Example 8, the oxycodone formulations provided an equivalent bioavailability of oxycodone in terms of $AUC_t$ and $AUC_{INF}$, relative to OxyContin® Tablets and relative to each other.

All three formulations have similar mean plasma oxycodone concentrations at 12 hours, suggesting that all three formulations show potential for being developed as a 12 hourly product.

Example 5 fasting was most similar to OxyContin® Tablets in terme of $AUC_t$, $AUC_{INF}$ and $C_{max}$.

EXAMPLE 20: A COMBINANON TAMPER RESISTANT PRODUCT

Co-encapsulation of extruded oxycodone multiparticulates and extruded naltrexone or naloxone multiparticulates can be used for a tamper resistant combination product.

Oxycodone multiparticulates and naltrexone multiparticulates as described in WO 03013433 may be filled into capsules using a single or dual stage filling process. The quantity of naltrexone multiparticulates which may be filled is 150 mg, containing 8 mg of naltrexone. The recommended fill weights of oxycodone multiparticulates to achieve oxycodone doses ranging from 10 mg to 40 mg are as follows (see also the following table):

1. 120 mg and 240 mg of 8.3% (w/w) drug loaded multiparticulates for oxycodone doses of 10 mg and 20 mg, respectively.
2a. 120 mg of 33.3% (w/w) drug loaded multiparticulates for an oxycodone dose of 40 mg or
2b. 160 mg of 25% (w/w) drug loaded multiparticulates for an oxycodone dose of 40 mg.

In addition, 5 mg and 80 mg oxycodone doses may also be considered, with respective capsule fill weights as follows:

1. 60 mg of 8.3% (w/w) drug loaded multiparticulates for an oxycodone dose of 5 mg.
2a. 240 mg of 33.3% (w/w) drug loaded multiparticulates for an oxycodone dose of 80 mg or
2b. 320 mg of 25% (w/w) drug loaded multiparticulates for an oxycodone dose of 80 mg.

For the drug load of 33.3% (w/w), the following trial formulations indicated 20.A and 20.B were prepared, where the weights are mg per unit dose:

|  | 20.A | 20.B |
|---|---|---|
| Oxycodone HCl | 40.0 | 40.0 |
| Eudragit RS PO | 67.0 | 67.0 |
| Stearyl Alcohol | 13.0 | 8.0 |
| Glyceryl behenate |  | 5.0 |
| Total | 120 | 120 |

These two formulations were initially manufactured for proof of principle for a higher strength product, and without Eudragit RL PO. The dissolution profiles from these formulations were slower than required and can be readily modified by the use of a water permeability modifier in accordance with the invention.

Capsule filling of the required proportions of oxycodone and naltrexone multiparticulates may be achieved using either a single stage process or preferably a dual stage filling process. In the single stage filling process, the respective proportions of multiparticulates may be preblended and filled into capsules either by manual or preferably automated process. By the preferred dual stage filling process, one type of multiparticulates can be filled in a first stage, either by manual or preferably automated processes. The second type of multiparticulates can then be filled in the second filling stage, again either by manual or preferably automated processes.

The theoretical fill weights for a range of capsule strengths based on drug loading are given in the following tables.

| oxycodone mg per capsule | oxycodone loading 8.3 % w/w | |
|---|---|---|
|  | oxycodone multi-particulates (mg) | oxycodone and naltrexoneØ multi-particulates (mg) |
| 10 | 120 | 270 (capsule Size 1) |
| 20 | 240 | 390 (capsule Size 0) |
| 40 | 480 | 630 (can not be filled) |
| 5+ | 60* | 210 (capsule Size 1) |
| 80+ | 960 | 1110 (can not be filled) |
| * weight below assumed minimum possible capsule fill weight.<br>+ Included as an illustration of possibilities, if lower or higher strengths in the range are required.<br>Ø 120 mg naltrexone multiparticulates + 20% coat. | | |

| oxycodone mg per capsules | oxycodone loading 25 % w/w | |
|---|---|---|
|  | oxycodone multi-particulates (mg) | oxycodone and naltrexoneØ multi-particulates (mg) |
| 10 | 40* | Low to fill. |
| 20 | 80 | 230 (capsule Size 1) |
| 40 | 160 | 310 (capsule Size 0) |
| 5+ | 20* | Low to fill |

(continued)

| oxycodone mg per capsules | oxycodone loading 25 % w/w | |
|---|---|---|
| | oxycodone multi-particulates (mg) | oxycodone and naltrexoneØ multi-particulates (mg) |
| 80+ | 320 | 470 (capsule Size OE) |
| * Weight below assumed minimum possible capsule fill weight. + Included is an illustration of possibilities, if lower or higher strengths in the range are required. Ø 120 mg naltrexone multiparticulates + 20% coat. | | |

Example 21: Alternate Cutter Procedure

For the Example, an alternate cutting procedure was employed. Extrudate emerges from the twelve orifices of the die-head shown-in-Figure of a Leistritz 18 extruder. A rotary cutter with two blades is used to cut the extruded mix as it emerges under pressure and still molten from the orifices of the die plate. The blades sweep over the surface of the die-head to pass the orifices. As they expand and cool, the cut extrudate particles tend to form rounded surfaces.

The following formulation was employed. This is outside the scope of the invention.

| Material | % w/w |
|---|---|
| Lactose anhydrous | 10.0 |
| Eudragit RS PO | 91.0 |
| Triethyl citrate | 10.0 |
| PEG 6000 | 6.0 |
| Magnesium Stearate | 4.5 |
| Total | 121.8 |

By appropriate adjustment of the extrusion parameters, including temperatures and rates of cooling, spherical or substantially spherical multiparticulates may be obtained.

**Claims**

1. Melt extruded controlled release multiparticulates which contain (a) oxycodone, (b) water-insoluble ammonium methacrylate copolymer, (c) plasticiser, (d) lubricant and (e) water permeability modifier, wherein said water permeability modifier is microcrystalline cellulose, croscarmellose sodium, crospovidone or sodium starch glycollate.

2. Multiparticulates according to claim 1, wherein the oxycodone is present as a pharmaceutically acceptable salt.

3. Multiparticulates according to claim 2, wherein the oxycodone is present as oxycodone hydrochloride.

4. Multiparticulates according to any of claims 1 to 3, wherein the plasticiser is chosen from cetyl alcohol, stearyl alcohol, cetostearyl alcohol, sorbitol, sucrose, high molecular weight polyethylene glycol, dibutyl sebacate, tributyl citrate, triethyl citrate, propylene glycol and low molecular weight polyethylene glycol.

5. Multiparticulates according to claim 4, wherein the plasticiser is stearyl alcohol.

6. Multiparticulates according to claim 4, wherein the plasticiser is a high molecular weight polyethylene glycol.

7. Multiparticulates according to any one of claims 1 to 6, wherein the lubricant is chosen from glyceryl behenate, talc and silicon dioxide.

8. Multiparticulates according to claim 7, wherein the lubricant is glyceryl behenate.

9. Multiparticulates according to any one of claims 1 to 6, wherein the lubricant is stearic acid or a stearate salt.

10. Multiparticulates according to any one of claims 1 to 9, wherein the water permeability modifier is microcrystalline cellulose.

11. Multiparticulates according to any one of claims 1 to 10, wherein the percentage amounts of the ingredients (a) to (e) are as given in the following table, based on the total weight of the five ingredients:

| | |
|---|---|
| oxycodone as hydrochloride | 3 to 50 |
| insoluble ammonium methacrylate copolymer | 25 to 85 |
| plasticiser | 1 to 30 |
| lubricant | 1 to 25 |
| water permeability modifier | 1 to 40 |

12. Muitiparticulates according to claim 11, wherein the percentage amounts of the ingredients (a) to (e) are as given in the following table, based on the total weight of the five ingredients:

| | |
|---|---|
| oxycodone as hydrochloride | 5 to 40 |
| insoluble ammonium methacrylate copolymer | 35 to 75 |
| plasticiser | 3 to 25 |
| lubricant | 2 to 25 |
| water permeability modifier | 1 to 30 |

13. Multiparticulates according to claim 11, wherein the percentage amounts of the ingredients (a) to (e) are as given in the following table, based on the total weight of the five ingredients:

| | |
|---|---|
| oxycodone as hydrochloride | 7.5 to 35 |
| insoluble ammonium methacrylate copolymer | 50 to 65 |
| plasticiser | 5 to 15 |
| lubricant | 2 to 25 |
| water permeability modifier | 1 to 20 |

14. A pharmaceutical composition in unit dose form comprising multiparticulates according to any preceding claim.

15. A pharmaceutical composition according to claim 14, wherein the unit dose provides a dose of oxycodone sufficient to provide analgesia to a human patient.

16. A pharmaceutical composition according to claim 15 which is bioequivalent to OxyContin® Tablets.

17. A pharmaceutical composition according to claim 15 or 16, wherein the sufficient dose of oxycodone is 5 to 400 mg.

18. A pharmaceutical composition according to claim 17, wherein the unit dose of oxycodone is 5 mg, 10 mg, 20 mg, 40 mg, 80 mg or 160 mg.

19. A pharmaceutical composition according to any of claims 14 to 18 in the form of a capsule with a fill of said multi-particulates.

20. A pharmaceutical composition according to claim 19, wherein the multiparticulates are filled into hard gelatin capsules each containing a unit dose.

21. A pharmaceutical composition according to claim 20, wherein the fill weight in the capsule is in the range 120 to 500 mg.

22. A pharmaceutical composition according to any of claims 14 to 21, which is intended for administration at intervals of 12 hours.

23. A pharmaceutical composition according to any one of claims 14, 15 and 17 to 21, which is intended for administration at intervals of 24 hours.

24. A pharmaceutical composition comprising multiparticulates according to any of claims 1 to 13 and multiparticulates of oxycodone antagonist.

25. A process for preparing multiparticulates as claimed in claim 1 which comprises preparing a blend which contains (a) oxycodone, (b) water-insoluble ammonium methacrylate copolymer, (c) plasticiser, (d) lubricant and (e) water permeability modifier, wherein said water permeability modifier is a microcrystalline cellulose, croscarmellose sodium, crospovidone or sodium starch glycollate; and extruding the blend.

26. A composition as defined in any one of claims 14 to 24 for use in medicine.

27. A composition as defined in any one of claims 14 to 24 for use in providing pain relief.

28. A composition as defined in any one of claims 14 to 24 for providing analgesia.

29. Multiparticulates as defined in any one of claims 1 to 13 for use in medicine.

30. Multiparticulates as defined in any one of claims 1 to 13 for use in providing pain relief.

31. Multiparticulates as defined in any one of claims 1 to 13 for providing analgesia.

32. Use of multiparticulates as defined in any one of claims 1 to 13 in the preparation of a composition for use in providing pain relief.

33. Use of multiparticulates as defined in any one of claims 1 to 13 in the preparation of a composition for providing analgesia.


**Patentansprüche**

1. Schmelzextrudierte Multipartikel mit kontrollierter Freisetzung, die enthalten: (a) Oxykodon, (b) wasserunlösliches Ammoniummethacrylat-Copolymer, (c) Weichmacher, (d) Gleitmittel und (e) Wasserdurchlässigkeitsmodifikator, wobei der Wasserdurchlässigkeitsmodifikator mikrokristalline Cellulose, Croscarmellose Natrium, Crospovidone oder Natriumstärkeglycolat ist.

2. Multipartikel nach Anspruch 1, wobei das Oxykodon als pharmazeutisch akzeptierbares Salz anwesend ist.

3. Multipartikel nach Anspruch 2, wobei das Oxykodon als Oxykodonhydrochlorid anwesend ist.

4. Multipartikel nach einem der Ansprüche 1 bis 3, wobei der Weichmacher unter Cetylalkohol, Stearylalkohol, Cetostearylalkohol, Sorbitol, Saccharose, Polyethylenglycol mit hohem Molekulargewicht, Dibutylsebacat, Tributylcitrat, Triethylcitrat, Propylenglycol und Polyethylenglycol mit niedrigem Molekulargewicht ausgewählt ist.

5. Multipartikel nach Anspruch 4, wobei der Weichmacher Stearylalkohol ist.

6. Multipartikel nach Anspruch 4, wobei der Weichmacher ein Polyethylenglycol mit hohem Molekulargewicht ist.

7. Multipartikel nach einem der Ansprüche 1 bis 6, wobei das Gleitmittel unter Glycerylbehenat, Talkum und Siliciumdioxid ausgewählt ist.

8. Multipartikel nach Anspruch 7, wobei das Gleitmittel Glycerylbehenat ist.

9. Multipartikel nach einem der Ansprüche 1 bis 6, wobei das Gleitmittel Stearinsäure oder ein Stearatsalz ist.

**10.** Multipartikel nach einem der Ansprüche 1 bis 9, wobei der Wasserdurchlässigkeitsmodifikator mikrokristalline Cellulose ist.

**11.** Multipartikel nach einem der Ansprüche 1 bis 10, wobei die Anteile der Bestandteile (a) bis (e) in Prozent den Angaben in der folgenden Tabelle entsprechen, bezogen auf das Gesamtgewicht der fünf Bestandteile:

| | |
|---|---|
| Oxykodon als Hydrochlorid | 3 bis 50 |
| unlösliches Ammoniummethacrylat-Copolymer | 25 bis 85 |
| Weichmacher | 1 bis 30 |
| Gleitmittel 1 bis | 25 |
| Wasserdurchlässigkeitsmodifikator | 1 bis 40. |

**12.** Multipartikel nach Anspruch 11, wobei die Anteile der Bestandteile (a) bis (e) in Prozent den Angaben in der folgenden Tabelle entsprechen, bezogen auf das Gesamtgewicht der fünf Bestandteile:

| | |
|---|---|
| Oxykodon als Hydrochlorid | 5 bis 40 |
| unlösliches Ammoniummethacrylat-Copolymer | 35 bis 75 |
| Weichmacher | 3 bis 25 |
| Gleitmittel | 2 bis 25 |
| Wasserdurchlässigkeitsmodifikator | 1 bis 30. |

**13.** Multipartikel nach Anspruch 11, wobei die Anteile der Bestandteile (a) bis (e) in Prozent den Angaben in der folgenden Tabelle entsprechen, bezogen auf das Gesamtgewicht der fünf Bestandteile:

| | |
|---|---|
| Oxykodon als Hydrochlorid | 7,5 bis 35 |
| unlösliches Ammoniummethacrylat-Copolymer | 50 bis 65 |
| Weichmacher | 5 bis 15 |
| Gleitmittel | 2 bis 25 |
| Wasserdurchlässigkeitsmodifikator | 1 bis 20. |

**14.** Pharmazeutische Zusammensetzung in Dosierungseinheitsform, die Multipartikel nach einem der vorstehenden Ansprüche aufweist.

**15.** Pharmazeutische Zusammensetzung nach Anspruch 14, wobei die Dosierungseinheit eine Oxykodon-Dosis liefert, die ausreicht, um bei einem menschlichen Patienten für Analgesie zu sorgen.

**16.** Pharmazeutische Zusammensetzung nach Anspruch 15, die bioäquivalent zu OxyContin®-Tabletten ist.

**17.** Pharmazeutische Zusammensetzung nach Anspruch 15 oder 16, wobei die ausreichende Oxykodon-Dosis 5 bis 400 mg beträgt.

**18.** Pharmazeutische Zusammensetzung nach Anspruch 17, wobei die Dosierungseinheit von Oxykodon 5 mg, 10 mg, 20 mg, 40 mg, 80 mg oder 160 mg beträgt.

**19.** Pharmazeutische Zusammensetzung nach einem der Ansprüche 14 bis 18 in Form einer Kapsel mit einer Füllung der Multipartikel.

**20.** Pharmazeutische Zusammensetzung nach Anspruch 19, wobei die Multipartikel in Hartgelatinekapseln eingefüllt werden, die jeweils eine Dosierungseinheit enthalten.

**21.** Pharmazeutische Zusammensetzung nach Anspruch 20, wobei das Füllgewicht in der Kapsel im Bereich von 120 bis 500 mg liegt.

**22.** Pharmazeutische Zusammensetzung nach einem der Ansprüche 14 bis 21, die zur Verabreichung in Intervallen von 12 Stunden vorgesehen ist.

**23.** Pharmazeutische Zusammensetzung nach einem der Ansprüche 14, 15 und 17 bis 21, die zur Verabreichung in Intervallen von 24 Stunden vorgesehen ist.

**24.** Pharmazeutische Zusammensetzung, die Multipartikel nach einem der Ansprüche 1 bis 13 und Multipartikel eines Oxykodon-Antagonisten aufweist.

**25.** Verfahren zur Herstellung von Multipartikeln nach Anspruch 1, das aufweist: Herstellen eines Gemischs, das (a) Oxykodon, (b) wasserunlösliches Ammoniummethacrylat-Copolymer, (c) Weichmacher, (d) Gleitmittel und (e) Wasserdurchlässigkeitsmodifikator enthält, wobei der Wasserdurchlässigkeitsmodifikator eine mikrokristalline Cellulose, Croscarmellose Natrium, Crospovidone oder Natriumstärkeglycolat ist; und Extrudieren des Gemischs.

**26.** Zusammensetzung wie in einem der Ansprüche 14 bis 24 definiert, für eine Verwendung in der Medizin.

**27.** Zusammensetzung wie in einem der Ansprüche 14 bis 24 definiert, zur Verwendung bei der Schmerzlinderung.

**28.** Zusammensetzung wie in einem der Ansprüche 14 bis 24 definiert, um Analgesie herbeizuführen.

**29.** Multipartikel wie in einem der Ansprüche 1 bis 13 definiert, zur Verwendung in der Medizin.

**30.** Multipartikel wie in einem der Ansprüche 1 bis 13 definiert, zur Verwendung bei der Schmerzlinderung.

**31.** Multipartikel wie in einem der Ansprüche 1 bis 13 definiert, um Analgesie herbeizuführen.

**32.** Verwendung von Multipartikeln wie in einem der Ansprüche 1 bis 13 definiert, für die Herstellung einer Zusammensetzung zur Verwendung bei der Herbeiführung von Schmerzlinderung.

**33.** Verwendung von Multipartikeln wie in einem der Ansprüche 1 bis 13 definiert, für die Herstellung einer Zusammensetzung, um Analgesie herbeizuführen.


**Revendications**

**1.** Formulations multiparticulaires extrudées à l'état fondu à libération contrôlée, qui contiennent (a) de l'oxycodone, (b) un copolymère de méthacrylate d'ammonium insoluble dans l'eau, (c) un agent plastifiant, (d) un lubrifiant et (e) un agent de modification de la perméabilité à l'eau, dans lesquelles ledit agent de modification de la perméabilité à l'eau est la cellulose microcristalline, la croscarmellose sodique, le crospovidone ou le glycolate d'amidon sodique.

**2.** Formulations multiparticulaires selon la revendication 1, dans lesquelles l'oxycodone est présente sous la forme d'un sel pharmaceutiquement acceptable.

**3.** Formulations multiparticulaires selon la revendication 2, dans lesquelles l'oxycodone est présente sous la forme de chlorhydrate d'oxycodone.

**4.** Formulations multiparticulaires selon l'une quelconque des revendications 1 à 3, dans lesquelles l'agent plastifiant est choisi parmi l'alcool de cétyle, l'alcool de stéaryle, l'alcool de cétostéaryle, le sorbitol, le saccharose, le polyéthylène glycol de haut poids moléculaire, le sébacate de dibutyle, le citrate de tributyle, le citrate de triéthyle, le propylène glycol et le polyéthylène glycol de faible poids moléculaire.

**5.** Formulations multiparticulaires selon la revendication 4, dans lesquelles l'agent plastifiant est l'alcool de stéaryle.

**6.** Formulations multiparticulaires selon la revendication 4, dans lesquelles l'agent plastifiant est un polyéthylène glycol de haut poids moléculaire.

**7.** Formulations multiparticulaires selon l'une quelconque des revendications 1 à 6, dans lesquelles le lubrifiant est choisi parmi le béhénate de glycéryle, le talc et le dioxyde de silicium.

8. Formulations multiparticulaires selon la revendication 7, dans lesquelles le lubrifiant est le béhénate de glycéryle.

9. Formulations multiparticulaires selon l'une quelconque des revendications 1 à 6, dans lesquelles le lubrifiant est l'acide stéarique ou un sel stéarate.

10. Formulations multiparticulaires selon l'une quelconque des revendications 1 à 9, dans lesquelles l'agent de modification de la perméabilité à l'eau est de la cellulose microcristalline.

11. Formulations multiparticulaires selon l'une quelconque des revendications 1 à 10, dans lesquelles les quantités en pourcentage des ingrédients (a) à (e) sont telles que données dans le tableau suivant, sur la base du poids total des cinq ingrédients:

| | |
|---|---|
| oxycodone comme chlorhydrate | 3 à 50 |
| copolymère de méthacrylate d'ammonium insoluble | 25 à 85 |
| agent plastifiant | 1 à 30 |
| lubrifiant | 1 à 25 |
| agent de modification de la perméabilité à l'eau | 1 à 40 |

12. Formulations multiparticulaires selon la revendication 11, dans lesquelles les quantités en pourcentage des ingrédients (a) à (e) sont telles que données dans le tableau suivant, sur la base du poids total des cinq ingrédients:

| | |
|---|---|
| oxycodone comme chlorhydrate | 5 à 40 |
| copolymère de méthacrylate d'ammonium insoluble | 35 à 75 |
| agent plastifiant | 3 à 25 |
| lubrifiant | 2 à 25 |
| agent de modification de la perméabilité à l'eau | 1 à 30 |

13. Formulations multiparticulaires selon la revendication 11, dans lesquelles les quantités en pourcentage des ingrédients (a) à (e) sont telles que données dans le tableau suivant, sur la base du poids total des cinq ingrédients:

| | |
|---|---|
| oxycodone comme chlorhydrate | 7,5 à 35 |
| copolymère de méthacrylate d'ammonium insoluble | 50 à 65 |
| agent plastifiant | 5 à 15 |
| lubrifiant | 2 à 25 |
| agent de modification de la perméabilité à l'eau | 1 à 20 |

14. Composition pharmaceutique sous la forme de dose unitaire comprenant les formulations multiparticulaires selon l'une quelconque des revendications précédentes.

15. Composition pharmaceutique selon la revendication 14, dans laquelle la dose unitaire fournit une dose d'oxycodone suffisante pour fournir l'analgésie à un patient humain.

16. Composition pharmaceutique selon la revendication 15, qui est l'équivalent biologique des comprimés d'OxyContin®.

17. Composition pharmaceutique selon la revendication 15 ou 16, dans laquelle la dose suffisante d'oxycodone est de 5 à 400 mg.

18. Composition pharmaceutique selon la revendication 17, dans laquelle la dose unitaire d'oxycodone est de 5 mg, 10 mg, 20 mg, 40 mg, 80 mg ou 160 mg.

19. Composition pharmaceutique selon l'une quelconque des revendications 14 à 18, sous la forme d'une capsule avec une charge desdites formulations multiparticulaires.

**20.** Composition pharmaceutique selon la revendication 19, dans laquelle les formulations multiparticulaires sont chargées dans des capsules de gélatine dure contenant chacune une dose unitaire.

**21.** Composition pharmaceutique selon la revendication 20, dans laquelle le poids de la charge dans la capsule se situe dans la plage de 120 à 500 mg.

**22.** Composition pharmaceutique selon l'une quelconque des revendications 14 à 21, qui est prévue pour l'administration à des intervalles de 12 heures.

**23.** Composition pharmaceutique selon l'une quelconque des revendications 14, 15 et 17 à 21, qui est prévue pour l'administration à des intervalles de 24 heures.

**24.** Composition pharmaceutique comprenant des formulations multiparticulaires selon l'une quelconque des revendications 1 à 13 et des formulations multiparticulaires d'antagoniste d'oxycodone.

**25.** Procédé de préparation des formulations multiparticulaires telles que revendiquées dans la revendication 1, qui comprend la préparation d'un mélange qui contient (a) de l'oxycodone, (b) un copolymère de méthacrylate d'ammonium insoluble dans l'eau, (c) un agent plastifiant, (d) un lubrifiant et (e) un agent de modification de la perméabilité à l'eau, dans lequel ledit agent de modification de la perméabilité à l'eau est la cellulose microcristalline, la croscarmellose sodique, le crospovidone ou le glycolate d'amidon sodique, et l'extrusion du mélange.

**26.** Composition telle que définie dans l'une quelconque des revendications 14 à 24 pour une utilisation en médecine.

**27.** Composition telle que définie dans l'une quelconque des revendications 14 à 24 pour une utilisation visant à procurer un soulagement de la douleur.

**28.** Composition telle que définie dans l'une quelconque des revendications 14 à 24 pour fournir une analgésie.

**29.** Formulations multiparticulaires telles que définies dans l'une quelconque des revendications 1 à 13 pour une utilisation en médecine.

**30.** Formulations multiparticulaires telles que définies dans l'une quelconque des revendications 1 à 13 pour une utilisation visant à procurer un soulagement de la douleur.

**31.** Formulations multiparticulaires telles que définies dans l'une quelconque des revendications 1 à 13 pour fournir une analgésie.

**32.** Utilisation des formulations multiparticulaires telles que définies dans l'une quelconque des revendications 1 à 13 dans la préparation d'une composition pour une utilisation visant à procurer un soulagement de la douleur.

**33.** Utilisation des formulations multiparticulaires telles que définies dans l'une quelconque des revendications 1 à 13 dans la préparation d'une composition pour fournir une analgésie.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9310765 A
- WO 9614058 A
- WO 02087512 A
- WO 0313433 A
- WO 03013433 A